# EUROPEAN PATENT SPECIFICATION

(11) **EP 0 881 899 B1**
(45) Date of publication and mention of the grant of the patent: **18.08.2004**
(21) Application number: 97903352.9
(22) Date of filing: 19.02.1997
(51) Int. Cl.: A61K 9/20

(54) **DOSAGE FORM OF IBUPROFEN**
DOSIERUNGSFORM VON IBUPROFEN
FORME PHARMACEUTIQUE D'IBUPROFENE

(30) Priority: 21.02.1996 GB 9603699
(43) Date of publication of application: 09.12.1998
(73) Proprietor: The Boots Company PLC, Nottingham, Nottinghamshire NG2 3AA (GB)
(72) Inventor: PRICE, Ian, Ashley, Nottingham NG2 3AA (GB)
(74) Representative: Frith, Richard William
(86) International application number: PCT/EP1997/000841
(87) International publication number: WO 1997/030699

(56) References cited:
- EP-A- 0 478 838
- EP-A- 0 607 467
- WO-A-89/02266
- WO-A-93/23026

## Description

This invention relates to a non-effervescent compressed solid dosage form for oral administration, to a process to make said dosage form and to its therapeutic utility.

Ibuprofen, namely 2-(4-isobutylphenyl)propionic acid, is a well known medicament with analgesic, anti-inflammatory and anti-pyretic properties. It is usually sold in the form of racemic ibuprofen (equal amounts of the S(+)-ibuprofen and R(-)-ibuprofen enantiomers). It may also be in the form of the purified form of either enantiomer, especially S(+)-ibuprofen which is acknowledged to be the active form of racemic ibuprofen. Ibuprofen is also available in salt form, for example the sodium salt of ibuprofen. Ibuprofen is available under prescription in the UK (eg Brufen (RTM)), primarily for the treatment of painful and anti-inflammatory disorders including rheumatoid arthritis, ankylosing spondylitis, osteoarthritis, postoperative pain, post partum pain and soft tissue injuries, generally at doses up to 3200mg per day. Ibuprofen is also available as a non-prescription drug in the UK (eg Nurofen (RTM)), primarily for the treatment of symptoms of pain and fever including headache, migraine, rheumatic pain, muscular pain, backache, neuralgia, dysmenorrhoea, dental pain and colds and flu, generally at doses up to 1200mg per day. A unit dose of ibuprofen or derivative thereof is generally equivalent to 200mg, 400mg, 600mg or 800mg racemic ibuprofen.

A major issue in connection with the above disorders is to improve the onset of action of ibuprofen, particularly in the treatment of pain. It is believed that rapid disintegration of a formulation releases the drug into the body quickly leading to a more rapid onset of therapeutic action compared with a standard dosage form. Accordingly, it is desired to produce a solid dosage form for oral administration adapted to disintegrate quickly in the gastro-intestinal tract. It is also preferred that the dosage form is manufactured by compression on standard tabletting machines with granulation and drying stages prior to tabletting optional. However, there are a number of formulation problems associated with providing a rapidly disintegrating solid dosage form containing an ibuprofen medicament. One problem is that, in order to achieve a therapeutic dose, solid compositions generally contain a high dose of drug, eg 200-800mg ibuprofen, which thus forms a considerable proportion of the dosage form, ie greater than 35% by weight. Thus, there is a problem to include the ibuprofen medicament, together with the excipients useful to formulate the tablet into a dosage form and the excipients useful to ensure rapid disintegration, but also to provide a tablet that is both not too large for patient consumption and can be manufactured according to standard processes. Furthermore, the solid dosage form must be sufficiently hard to withstand the rigours of the manufacturing process, for example as encountered during the stage of film coating in a perforated rotating drum, and packaging etc, but must have appropriate disintegration characteristics to ensure rapid release of the drug from the formulation. Another desirable feature is that a composition comprising a mixture of the desired ingredients is capable of being compressed without sticking to the punches of the tabletting machine.

Previously, it has been found that a slight increase in the tabletting compaction pressure, in order to improve the hardness properties, led to a significant increase in the disintegration time of the resulting tablet. Thus, when compressing ingredients, it was difficult to use standard tabletting machine compaction pressures to arrive at an appropriate tablet disintegration time and maintain an acceptably sized tablet of sufficient hardness.

German Patent Application 3922441A seeks to improve the tablettability of ibuprofen compositions and discloses that this may be achieved by converting ibuprofen wholly or partially into its calcium salt and using these for tabletting. It is said that the compositions may optionally contain ibuprofen, S(+)-ibuprofen or their ammonium, sodium or potassium salts. The calcium salt and the optional other ibuprofen actives may be incorporated into the tablet as separately produced compounds or the salts may be formed in-situ during the tablet preparation method through the reaction between ibuprofen (an acidic drug) with a solution or suspension of a reactant comprising one or more of CaO, Ca(OH)₂, CaCO₃, NaOH, KOH, NH₄OH, Na₂CO₃, NaHCO₃, K₂CO₃, KHCO₃, (NH₄)₂CO₃, NH₄HCO₃ (in an amount of 25% to 110% of the equivalent quantity of ibuprofen). The mixture obtained is then granulated, dried if appropriate, and then tabletted after the optional incorporation of other excipients. The specification comments that depending on the proportions of other salts used with the calcium salt, the ammonium and alkali salts improve the solubility of the calcium salt-containing compositions and thus control the bioavailability, but they also increase the hygroscopicity and stickiness. These are both undesirable characteristics for optimum tabletting. This disclosure does not seek to improve the disintegration time. WO Patent Application 9323026 also relates to the inclusion of calcium compounds into 2-arylpropionic compositions to improve tablettability. The 2-arylpropionic acid (100 parts by weight) is dry mixed with the calcium compound (about 50-500 parts by weight). However, due to the amounts of these ingredients which are necessary, the calcium compound must be the major component of the compressible filler.

We have now found that by incorporating an alkali metal carbonate or bicarbonate in the composition for compression, a solid dosage form of acceptable size containing an ibuprofen medicament can be produced which has a rapid disintegration time and satisfactory hardness. The present invention is based on the discovery that the addition of an alkali metal carbonate or bicarbonate enhances the compressibility of a composition containing a compressible filler in combination with a disintegrant component leading to a solid dosage form with valuable hardness and disintegration characteristics. The composition comprises a solid non-effervescent compressed dosage form comprising a racemic ibuprofen medicament and a carrier material comprising a compressible filler component combined with a disintegrating component wherein the ibuprofen medicament is present to an extent of 35% or more by weight of the dosage form, and the carrier material further includes a solid alkali metal carbonate or bicarbonate present in an amount of 3-20% by weight of the dosage form, the dosage form having a crushing strength in the range 63,7-147 N (6,5-15 Kp) and a disintegration time of less than 10 minutes as measured by the method described in the European Pharmacopoeia 1986, Ref. V.5.1.1.

The disclosure in German Patent Application 3922441A of compositions containing the calcium salt optionally with an ibuprofen sodium or potassium salt, formed in-situ in the presence of a liquid during tablet formation, are outside the scope of the present invention.

European Patent Application 607467 relates to the preparation of pellets containing a very high content of S(+)-ibuprofen and to the production of tablets comprising the pellets mixed with conventional auxiliary tabletting material. It discloses that the incorporation of small amounts of a basic salt or base (including sodium carbonate) in the pellets allows the preparation of pellets which contain 90% by weight or greater S(+)-Ibuprofen and which release the active very rapidly. The pellets can be prepared by spraying the mixture containing S(+)-ibuprofen and at least one adjunct with a basic inorganic salt in aqueous solution or with a dilute alkali metal hydroxide solution.

In one aspect, the present invention provides a solid non-effervescent compressed dosage form comprising a racemic ibuprofen medicament and a carrier material comprising a compressible filler component combined with a disintegrating component wherein the ibuprofen medicament is present to an extent of 35% or more by weight of the dosage form, characterised in that the carrier material includes a solid alkali metal carbonate or bicarbonate present in an amount of 3-20% by weight of the dosage form, said solid alkali metal carbonate or bicarbonate being in homogeneous admixture with said ibuprofen medicament and said compressible filler component combined with disintegrating component, such that the dosage form has a crushing strength in the range 63,7-147 N (6,5-15 Kp) and a disintegration time of less than 10 minutes as measured by the method described in the European pharmacopoeia 1986, Ref V.5.1.1, provided that the ibuprofen medicament does not contain a calcium salt of ibuprofen in combination with an alkali metal salt of ibuprofen.

In a further aspect, the present invention provides the use of an alkali metal carbonate or bicarbonate in a carrier material including a compressible filler component combined with a disintegrating component, said carrier material being arranged for admixture with a racemic ibuprofen medicament under substantially dry conditions and then for compression into a solid non-effervescent dosage form wherein said ibuprofen medicament comprises 35% or more by weight of the dosage form, said alkali metal carbonate or bicarbonate is present to an extent of 3-20% by weight of the dosage form, and said dosage form has a crushing strength in the range 63,7-147N (6,5-15Kp) and a disintegration time of less than 10 minutes as measured by the method described in the European pharmacopoeia 1986, Ref V.5.1.1.

The term "ibuprofen medicament" covers ibuprofen, salts, hydrates and other derivatives.

Crushing strength is a measure of the hardness of a compressed dosage form. It represents the pressure required to break the tablet. The crushing strength of the solid dosage form may be measured by any machine adapted for this purpose, ie by squeezing the dosage form between two jaws and measuring the force required to break the tablet diametrically. Suitable Crushing Strength Testers are available from Monsanto, Erweka and Schleuniger (manual or automatic operation). The disintegration time represents the time taken for the tablet to disintegrate in an aqueous medium under the test defined in the European Pharmacopoeia 1986 Ref V.5.1.1 (updated 1995).

Alkali metal carbonates and bicarbonates are not normally used as compressible materials. It was not expected that replacing a proportion of a compressible filler component in the composition with a portion of substantially incompressible alkali metal carbonate or bicarbonate would lead to a solid dosage form having both good crushing strength properties and good disintegration properties. It was also found that other soluble materials such as lactose, sucrose, mannitol, sodium citrate and sodium chloride did not yield tablets having the combination of satisfactory compressibility, crushing strength and disintegration properties and acceptable size, as is achieved by the use of the alkali metal carbonates or bicarbonates in a dosage form according to the present invention.

Alkali metal carbonates and bicarbonates are soluble materials which have previously been proposed for use in effervescent tablets, for example to react with the acid component in an effervescent couple (see for example WO 94/10994) or to prevent initiation of the effervescent reaction eg during storage. Effervescent tablets disintegrate by means of the reaction between acid and base particularly in the presence of water leading to the production of carbon dioxide. The system of disintegration of non-effervescent dosage forms according to the present invention, which are arranged to be swallowed and for which an effervescent reaction is not desired, is different to that of effervescent systems. The present dosage form does not contain any soluble acidic component with which the alkali metal carbonate or bicarbonate could react in an effervescent reaction.

Sodium bicarbonate is also known for use as an antacid and has previously been combined with ibuprofen in a tablet formulation for its antacid properties, eg Japanese Patent Application 63198620A. However, this document does not provide a disclosure relating to the incorporation of ibuprofen and sodium bicarbonate in a tablet with a compressible filler component combined with a disintegrating component or the formation of solid dosage forms having the crushing strength and disintegration properties that are characteristic of the present invention.

Sodium bicarbonate has also been proposed for use in a water-soluble composition which forms an acceptably-tasting drink product comprising ibuprofen (33-46% w/w), L-arginine (34-51%) and sodium bicarbonate (9-29%) (US 4834966). However, this disclosure does not disclose the other formulation ingredients useful to provide the crushing strength and disintegration characteristics of the present invention.

US 4873231 relates to decreasing the toxicity of an ibuprofen salt by combining the salt with from one to five molar excess of a bicarbonate or carbonate. Example 13 discloses that sodium ibuprofen is pressed into a tablet with one equivalent of sodium or potassium bicarbonate to provide a dosage of 200 or 400mg ibuprofen. It gives no further details of the formulation and therefore does not provide an enabling disclosure concerning the production a solid dosage form having the crushing strength and disintegration properties which characterises the present invention.

European Patent Application 418043A discloses that although the compounds selected from alkali metal bicarbonates, alkali metal monohydrogen phosphates and alkali metal tribasic citrates can be used to mask the taste of a water-soluble ibuprofen salt in solution, other materials including alkali metal carbonates cannot be used, because, in potential taste-masking amounts, the resultant aqueous solution has an unacceptably high pH for oral administration. The compositions used therein will usually be in the form of a free-flowing powder suitably contained in unit dose sachets. However, it is also disclosed that the composition could be in any other form such as a water-soluble tablet suitable for dissolution in water which can include a small amount of an effervescent couple to assist dispersion of the tablet on addition to water. However, there is no disclosure of a non-effervescent solid dosage form characterised by the crushing strength and disintegration properties according to the present invention.

The present invention allows any racemic ibuprofen medicament to be formulated into a solid dosage form using a carrier material common to all ibuprofen medicaments. Due to the different properties of the different ibuprofen medicaments, such as the melting point, the crystal form, particle size, the yield pressure etc, it is difficult to find a common carrier material which allows all forms of racemic ibuprofen to be compressed into a solid dosage form. Accordingly, where prior art disclosures particularly relate to formulation characteristics required of an ibuprofen dosage form and/or to compression into a solid dosage form, in many cases the disclosure relates particularly either to ibuprofen or to an ibuprofen salt. For example, European Patent Application 298666A, WO 90/08542, WO 89/02266 and US Patent 4609675, all relate to directly compressible formulations containing ibuprofen as the active ingredient, but do not extend their disclosures to salts. Thus, it is a particular advantage that the dosage form according to the invention may include both ibuprofen and salts thereof, particularly salts such as the sodium salt, where compression into a dosage form is particularly difficult.

The alkali metal carbonate or bicarbonate enhances the compressibility of the compressible filler in combination with the ibuprofen medicament. Thus, the use of an alkali metal carbonate or bicarbonate allows a reduction in the amount of compressible filler component that would normally be required in a composition to achieve satisfactory compressibility. This is of advantage as ibuprofen medicaments are usually administered in large doses. Thus minimising the amount of formulation excipients is valuable as it allows an acceptably sized dosage form to be produced. In accordance with the invention, the total amount of the compressible filler and alkali metal carbonate or bicarbonate that can be used is less than the amount of compressible filler component combined with a disintegrating component that would be required in the absence of the alkali metal carbonate or bicarbonate to achieve a dosage form with satisfactory hardness and disintegration characteristics.

The solid dosage forms according to the invention are adapted for direct administration to a patient to obtain the desired therapeutic effect. They are not intended to be dissolved or dispersed in water prior to administration. Furthermore, the compressed dosage forms according to the present invention need no further processing after compression of a composition comprising a mixture of the ingredients to produce a solid dosage form.

The ibuprofen molecule exists in two enantiomeric forms and the term ibuprofen medicament as used herein is intended to embrace a 1:1 mixture of enantiomers which is herein referred to as racemic ibuprofen. The ibuprofen medicament may be also present in the form of any salt or other derivative of ibuprofen. If necessary, the ibuprofen medicament may comprise one or more ibuprofen active ingredients. However, we prefer that the ibuprofen medicament comprises a single ibuprofen active ingredient. The ibuprofen medicament may also be present in different degrees of hydration. The present invention applies to both anhydrous and hydrated forms, for example the monohydrate or the dihydrate. The most stable anhydrous or hydrated form will generally be used. Preferably, the ibuprofen medicament is in the form of a salt of racemic ibuprofen. Representative examples include alkali metal salts, for example the sodium or potassium salts of ibuprofen; alkaline earth metal salts, eg the calcium or magnesium salts of ibuprofen; metal salts, eg the aluminium salt of ibuprofen; amino acid salts for example the lysine or arginine salts of ibuprofen; or amine salts, eg the meglumine salt of ibuprofen. Preferably the ibuprofen medicament is a single salt selected from alkali metal salts, amino acid salts and amine salts. Greater advantages are obtained in accordance with the present invention by the use of soluble salts of ibuprofen, for example the alkali metal salts such as sodium and potassium, as these materials are poorly compressible. For example, the sodium salt is a flaky, soft and sticky material. It does not lend itself to formulation into a dosage form as it is particularly difficult to compress. It is also difficult to pre-granulate the sodium salt prior to compression with other excipients into tablets. It thus usually requires an initial treatment stage such as milling, in order to form satisfactory tablets. However, no such pre-treatment of the sodium salt is required in accordance with the present invention. It is thus a further advantage to use sodium ibuprofen taken from a bulk production process to produce the raw material. These soluble ibuprofen salts also have the advantage that, as they are more soluble in an aqueous medium, on release from the formulation they have improved absorption, thus leading to an improved onset of action compared to the substantially insoluble forms of ibuprofen. The sodium salt of racemic ibuprofen is particularly preferred. It has been found that the dihydrate of the sodium salt of racemic ibuprofen is a particularly stable hydrated form, accordingly we prefer to use the sodium salt dihydrate in a compressed dosage form according to the present invention.

The particle size of the ibuprofen medicament should be such as to facilitate the manufacturing process, for example to permit flow during the manufacturing process and thus aid the compression process. Accordingly, preferably it has a median particle size in the range 25-600µm, preferably 50-300µm, most preferably 150-250µm.

It is generally desired to have as high a proportion of ibuprofen medicament in the dosage form as possible to reduce the size of the solid dosage form. Representative dosage forms generally comprise ibuprofen medicament to an extent to give 35-90% ibuprofen by weight of the formulation, preferably 35-75% by weight, more preferably 40-60% by weight and most preferably 45-55% by weight. Unit dosages may comprise ibuprofen medicament to an extent of 50mg, 100mg, 150mg, 200mg, 250mg, 300mg, 350mg, 400mg, 500mg, 600mg and 800mg. Where salts or other derivatives are employed, usually the precise unit doses are chosen to give the equivalent ibuprofen doses set out above, for example 256mg of the sodium salt dihydrate or 342mg of the dl lysine salt provides an equivalent dose to 200mg ibuprofen.

The alkali metal carbonate or bicarbonate aids the formation of a solid dosage form having the crushing strength and disintegration characteristics outlined above. The alkali metal carbonate or bicarbonate is suitably included in the dosage form in solid form. It is not necessary to dissolve it in a solvent, eg water, for a granulation step before compression into a solid dosage form. The properties of crushing strength and disintegration of the dosage form are achieved by the presence of the solid alkali metal carbonate or bicarbonate in homogenous admixture with the ibuprofen medicament and compressible filler with disintegrating component. It is particularly desired that the particles of the ibuprofen medicament and alkali metal carbonate or bicarbonate are intimately mixed.

The alkali metal carbonate or bicarbonate used in accordance with the present invention may suitably comprise sodium carbonate or bicarbonate or potassium carbonate or bicarbonate either alone or mixed together. Preferably, the alkali metal comprises sodium, thus sodium bicarbonate and sodium bicarbonate are preferred ingredients. The alkali metal carbonates may be supplied anhydrous or in varying degrees of hydration for example the monohydrate and decahydrate. Both these forms may be used. However, we prefer to use the anhydrous form. The preferred alkali carbonate for use in accordance with the present invention is thus anhydrous sodium carbonate.

The alkali metal carbonate or bicarbonate is present to aid the formation of the ibuprofen medicament dosage form and to provide a solid dosage form having a crushing strength in the range 63,7-147 N (6,5 - 15 Kp) and a disintegration time of less than 10 minutes. The alkali metal carbonate or bicarbonate is present in an amount of 3-20% by weight of the dosage form, preferably 4-16% by weight, more preferably 5-15% by weight and most preferably 6-10% by weight of the dosage form. The alkali metal carbonate or bicarbonate preferably has a particle size in the range of 25-600µm, more preferably 50-100µm. In preferred dosage forms the weight of sodium carbonate or bicarbonate to ibuprofen medicament is in the range 1:2 to 1:10 parts by weight. In a particularly preferred aspect of the invention the dosage form is in the form of a directly compressed tablet comprising 40-85% w/w sodium salt of ibuprofen and 5-15% w/w sodium carbonate or bicarbonate.

The carrier material forms suitably up to 65% by weight of the dosage form. Preferred dosage forms include 25-65% by weight carrier material, more preferably 40-60% by weight and most preferably 45-55% by weight carrier material. In a more preferred dosage form, the ratio of ibuprofen medicament to the carrier material is in the range 2:1 to 1:2 parts by weight and the carrier material comprises 5-20% w/w sodium carbonate or bicarbonate.

The carrier material comprises a compressible filler component which is used in a sufficient amount together with the alkali metal carbonate or bicarbonate to ensure that the composition containing the ibuprofen medicament is capable of being formed, preferably by direct compression, into a solid dosage form having a crushing strength in the range 63,7-147 N ( 6,5-15 Kp) and a disintegration time of less than 10 minutes. The ingredients are usually compressed from a dry powder mixture. The mixture may contain a pre-granulated product, eg formed by wet or dry granulation and optionally containing the ibuprofen medicament, and the dry granule produced may be combined with other dry powder ingredients, as necessary, and compressed into a solid dosage form. Usually, in any wet pre-granulation stage, the ibuprofen medicament would be present in the granule. The alkali metal carbonate or bicarbonate would be added to the formed granule with optional other excipients, such as a lubricant, prior to compression. However, preferably no liquid (ie water) is added to the formulation in any optional pre-granulation stage or prior to compression. It will also be appreciated that a directly compressible formulation has advantages as it represents a more efficient tabletting process, namely just mixing the ingredients and then compressing them, thus alleviating the need for the intermediate granulation and drying steps necessary in other tabletting procedures.

The compressible filler component is suitably present to an extent of 10-50% by weight of the dosage form, preferably 20-50% by weight of the dosage form, more preferably 27-45% by weight, most preferably 30-40% by weight of the dosage form. The ratio of alkali metal carbonate or bicarbonate to compressible filler component is preferably in the range 2:1 to 1:10 parts by weight.

Examples of the compressible filler component include one or more of cellulose derivatives, starch and derivatives thereof (eg pre-gelatinised starch), soluble sugars (eg lactose, sucrose, dextrin), sodium chloride, calcium phosphate, calcium sulphate, mannitol, sorbitol, cyclodextrin and maltodextrin. Preferably the compressible filler component comprises a cellulose derivative. Examples of suitable cellulose derivatives include methylcellulose, hydroxymethylcellulose, hydroxyethylcellulose, hydroxypropylcellulose, hydroxypropylmethylcellulose, hydroxypropylmethylcellulose phthalate and micro-crystalline cellulose. The preferred cellulose derivative used in accordance with the present invention is micro-crystalline cellulose. Further preferably, the cellulose derivative has a particle size above 100µm, preferably in the range 100-150µm.

In preferred dosage forms the cellulose derivative forms 50-100% by weight of the compressible filler component, more preferably 70-100% and most preferably 90-100% by weight of the compressible filler component. The remainder of the compressible filler component may be formed by other fillers known in the art including those listed above. Preferred compressible filler components comprise one or more of microcrystalline cellulose, lactose and mannitol. In a preferred aspect of the present invention, where the compressible filler component comprises 50-100% by weight of a cellulose derivative, the ratio of alkali metal carbonate or bicarbonate to cellulose derivative is suitably in the range of 2:1 to 1:10, more preferably 1:1 to 1:9 and especially 1:3 to 1:8 parts by weight. In a further preferred aspect the combined weight ratio of the cellulose derivative and alkali metal carbonate or bicarbonate to the ibuprofen medicament is 1:10 to 2:1 parts by weight, more preferably 1:4 to 2:1 parts by weight, most preferably 1:1 to 1:2 parts by weight.

The compressible filler component is combined with a disintegrating component. Examples of disintegrating components include one or more of wheat starch, maize starch, potato starch, sodium starch glycollate, low-substituted hydroxypropylcellulose, alginic acid, cross-linked polyvinylpyrrolidone, magnesium aluminium silicate and croscarmellose sodium. Preferred disintegrants comprise one or more of croscarmellose sodium and sodium starch glycollate. Such disintegrating agents, if used, may form up to 15% by weight of the dosage form, for example 1-10% by weight, preferably 5-15% by weight of the dosage form. Some compressible filler components have disintegrant properties, for example microcrystalline cellulose and/or hydroxypropylmethyl cellulose and therefore a discrete disintegrant material is not necessary as the compressible filler component is thus combined with a disintegrating component. However, we prefer to use a compressible filler component (which may have disintegrant properties) and a discrete disintegrant component which are separate components mixed into the composition.

In a particularly preferred dosage form the carrier material comprises 8-80% by weight compressible filler component (more preferably 50-75% by weight), 8-40% by weight alkali metal carbonate or bicarbonate (more preferably 10-20% by weight), 10-20% by weight disintegrant (more preferably 12-18% by weight). Especially preferred is a carrier material comprising 50-75% microcrystalline cellulose, 12-18% croscarmellose sodium and 8-20% sodium carbonate or bicarbonate. Another preferred carrier material comprises 45 to 60% by weight microcrystalline cellulose, 2 to 10% by weight croscarmellose sodium and 2 to 20% by weight sodium carbonate or bicarbonate. Desirably the ratio of the compressible filler to the alkali metal carbonate or bicarbonate to the disintegrant component is 1-9:1:0.5-2 parts by weight, preferably 2.5-6:1:0.8-1.4 parts by weight.

The compressed dosage form may also comprise one or more inert diluents (which are not characterised by the property of compressibility) as desired by the person skilled in the art. The inert diluent may be present up to an extent of 20% by weight of the formulation, suitably 0-10% by weight.

The solid dosage form may also include a flow aid such as talc or colloidal silicon dioxide, which may preferably be used to an extent of up to 4% by weight of the formulation, for example 0.5-2.0% by weight of the formulation. Lubricants such as stearic acid, sodium lauryl sulphate, polyethylene glycol, hydrogenated vegetable oil, calcium stearate, sodium stearyl fumarate or magnesium stearate may also be included in the dosage form. These may be used to an extent of up to 4% by weight of the dosage form, for example 0.5-2% by weight of the dosage form. Anti-adherents such as talc may further be included in an amount of up to 4% by weight of the dosage form, for example 0.5-2% by weight of the dosage form.

A solid dosage of the invention may be coated, eg with a sugar or film coating which has minimal effect on the disintegration time. A preferred solid dosage form of the present invention, ie a tablet, is film coated, such as by spraying tablets with a solution comprising hydroxypropylmethylcellulose and a plasticiser such as propylene glycol, polyethylene glycol and/or talc in one or more coatings.

A preferred dosage form comprises:-
(a) 40-60% by weight sodium salt of ibuprofen (more preferably 45-55% by weight);
(b) 20-50% by weight of a compressible filler, eg micro-crystalline cellulose (more preferably 30-40% by weight);
(c) 4-16% by weight sodium carbonate or sodium bicarbonate (more preferably 5-10% by weight);
(d) Up to 10% by weight of a disintegrant, eg croscarmellose sodium or sodium starch glycollate (more preferably 5-10% by weight);
(e) Up to 4% by weight of a lubricant, eg stearic acid (more preferably 0.5-2.0% by weight); and
(f) Up to 2% by weight of a flow aid, eg colloidal silicon dioxide (more preferably 0.5-1% by weight).

In a further preferred dosage form the ratio of ibuprofen medicament to carrier material is in the range 1:2 to 2:1 parts by weight, preferably 2:3 to 3:2 parts by weight, and the ratio of the cellulose derivative compressible filler component to alkali metal carbonate or bicarbonate is 9:1 to 1:1, preferably 5:1 to 3:1 parts by weight.

A solid dosage form produced in accordance with the present invention may be compressed, preferably directly compressed, to have a crushing strength in the range of 63,7-147 N (6,5-15 Kp), more preferably 78,4-117,6 N (8-12 Kp). This can be achieved, for example, using standard single punch or rotary tabletting machines having a compression force in the range 100-140MPa.

It will be appreciated by the person skilled in the art that due to the different excipients used in the formulation and varying amounts thereof that for any compression pressure, different formulations will have different crushing strengths and disintegration times. Preferred dosage forms exhibit a crushing strength of 63,7-147 N (6,5-15 Kp) and a disintegration time of less than 10 minutes at a compression force above 80MPa. More preferred formulations exhibit a crushing strength of 63,7-147 N (6,5-15 Kp) and a disintegration time of less than 10 minutes when compressed at a compression force in the range 100-140MPa such as by a standard tabletting machine, eg a rotary tabletting machine. Such compression pressures include, 110MPa, 120MPa and 130MPa. Especially preferred dosage forms exhibit a crushing strength of 63,7-147 N (6,5-15 Kp) and a disintegration time of less than 10 minutes when compressed at all pressures in the range 100-140MPa.

As disclosed hereinabove, it is necessary to have a dosage form of appropriate crushing strength. This is necessary so that the dosage form retains its integrity and does not crumble and/or break-up during the manufacturing process, the packaging process and transit of the packaged product. However, it is also necessary to ensure that the dosage form is not too hard that the drug cannot be released from the formulation quickly. Preferred dosage forms have a crushing strength in the range 68,6-117,6 N (7-12 Kp), more preferably 78,4-117,6 N (8-12 Kp). Preferably the dosage form has a crushing strength in the range 78,4- 117,6 N (8-12 Kp) at a compression force in the range 100-140MPa.

The disintegration time of the tablet formed in accordance with the present invention is less than 10 minutes as measured by the method described in the European Pharmacopoeia 1986, Ref V.5.1.1 (updated 1995) (A. Disintegration Test for Tablets and Capsules). Preferred disintegration times are less than 6 minutes (eg 1-6 minutes), more preferably less than 5 minutes (eg 1-5 minutes) and most preferably 3 minutes or less (eg 1-3 minutes).

The dosage forms according to the present invention may or may not be water-soluble. We have found that water-solubility of the dosage form is not crucial. Some of the materials found to be most useful in accordance with the present invention are insoluble. Accordingly, if one or more materials is insoluble, the dosage form is water-insoluble and this represents a preferred dosage form.

The dosage forms formed in accordance with the present invention are prepared by compression. The carrier material is combined with the ibuprofen medicament and compressed (preferably directly compressed) into a solid dosage form. The final stage of producing the solid dosage form (eg compression) may be preceded by a pre-granulation stage such as initial wet-granulation or initial dry granulation. In the wet granulation stage the ibuprofen medicament is generally pre-granulated with a binder, such as polyvinylpyrrolidone in a solvent, such as water or a hydrocarbon solvent and then the granules are dried. The granulated material is then mixed with other excipients as necessary and formed into a solid dosage form according to the invention. In any initial pre-granulation stage however, there is no requirement to add a solvent (eg water) at any stage during the manufacturing process and therefore, in a preferred embodiment of the invention, no drying stage is necessary. In a dry pre-granulation stage, certain of the components may be compressed together such as by roller compaction or slugging, and the granules are then mixed with the remaining excipients and compressed into a solid dosage form. The dosage forms may also be formed by sieving powdered ingredients into a container and then blending all of the ingredients to prepare a homogeneous mixture. The mixture may then be directly compressed to form tablets. This process forms a further aspect of the invention.

Thus, there is provided a process to prepare a non-effervescent solid dosage form comprising a racemic ibuprofen medicament present to an extent of 35% or more by weight of the dosage form characterised by combining a carrier material comprising a compressible filler component combined with a disintegrating component and 3-20% by weight of the dosage form of an alkali metal carbonate or bicarbonate with the ibuprofen medicament to form a homogeneous solid mixture under substantially dry conditions optionally with other tabletting excipients and compressing the mixture into one or more solid dosage forms having a crushing strength in the range 63,7-147 N (6,5-15 Kp) and a disintegration time of less than 10 minutes

In a more preferred process, the dosage form is prepared by direct compression of a powder mixture of the ingredients and does not include any pre-granulation stage. In such a process the ibuprofen medicament may be combined with the compressible filler component, a discrete disintegrant component and the alkali metal carbonate or bicarbonate. The other optional carrier excipients, such as a flow aid and a lubricant, may also be added and mixed so that all the powder particles are in intimate admixture, and finally the mixture is directly compressed into a solid dosage form according to the present invention.

In a preferred process, there is provided a dosage form comprising the sodium salt of ibuprofen together with a carrier material comprising microcrystalline cellulose and sodium carbonate or bicarbonate.

In therapeutic use the dosage forms of the present invention are administered orally, thus the therapeutic dosage forms are presented in solid dosage form, preferably as a tablet. The dosage forms may be coated with a sugar or film coating, which dissolves substantially immediately the dosage form comes into contact with an aqueous medium. The composition may also be compressed onto a solid core of another material to form a solid formulation with an quick release outer coating. Alternatively, the compressed composition may be present in one or more layers of a multi-layer solid dosage form. In such formulations the remaining layers or core may comprise standard excipients to provide conventional, fast or slow release and are well within the knowledge of a person skilled in the art (eg, see Remington's Pharmaceutical Sciences, 17th Edition, Ed Gennaro et al).

Thus, in a further preferred aspect the invention also provides a solid formulation having a layer comprising a composition comprising a racemic ibuprofen medicament together with a carrier material, the racemic ibuprofen medicament being present to an extent of 35% or more by weight of the composition and the carrier material comprising a compressible filler component combined with a disintegrating component characterised in that the carrier material comprises a solid alkali metal carbonate or bicarbonate present in an amount of 3-20% by weight of the composition, said solid alkali metal carbonate or bicarbonate being in homogeneous admixture with said ibuprofen medicament and said compressible filler component combined with disintegrating component such that the composition is capable of compression to provide a layer having a crushing strength in the range 63,7-147 N (6,5-15 Kp) and a disintegration time of less than 10 minutes.

The dosage forms of the present invention may, if desired, include other compatible pharmacologically active ingredients (for example centrally acting analgesics, eg codeine) and/or enhancing agents. Thus, for example, the dosage form may include any ingredient commonly used in a cough, cold or 'flu remedy, for example caffeine or another xanthine derivative, and/or another analgesic, and/or a skeletal muscle relaxant, and/or an antihistamine, and/or a decongestant, and/or a cough suppressant and/or an expectorant.

Suitable antihistamines include acrivastine, astemizole, azatadine, azelastine, bromodiphenhydramine, brompheniramine, carbinoxamine, cetirizine, chlorpheniramine, cyproheptadine, dexbromopheniramine, dexchlorpheniramine, diphenhydramine, ebastine, ketotifen, lodoxamide, loratidine, levocabastine, mequitazine, oxatomide, phenindamine, phenyltoloxamine, pyrilamine, setastine, tazifylline, temelastine, terfenadine, tripelennamine or triprolidine. Preferably non-sedating antihistamines are employed. Suitable cough suppressants include caramiphen, codeine or dextromethorphan. Suitable decongestants include pseudoephedrine, phenylpropanolamine and phenylephrine. Suitable expectorants include guaifenesin, potassium citrate, potassium guaiacolsulphonate, potassium sulphate and terpin hydrate.

Ibuprofen and its derivatives are primarily anti-inflammatory, analgesic and anti-pyretic agents but have also been proposed for other therapeutic uses, including the treatment of periodontal bone loss, pruritus and Alzheimer's disease. The dosage forms of the present invention are therefore indicated for use in the treatment of all therapeutic uses for which ibuprofen is effective, including rheumatoid arthritis, osteoarthritis, ankylosing spondylitis, seronegative arthropathies, periarticular disorders and soft tissue injuries. They may also be used in the treatment of postoperative pain, postpartum pain, dental pain, dysmenorrhoea, headache, migraine, rheumatic pain, muscular pain, backache, neuralgia and/or musculoskeletal pain or the pain or discomfort associated with the following: respiratory infections, colds or influenza, gout or morning stiffness.

In a further aspect, the present invention provides the use of a non-effervescent compressed solid dosage form comprising 35% or more by weight of a sodium salt of racemic ibuprofen together with a carrier material comprising a compressible filler component combined with a disintegrating component and an alkali metal carbonate or bicarbonate present in an amount of 3-20% by weight of the dosage form, the dosage form having a crushing strength in the range 63,7-147 N (6,5-15 Kp) and a disintegration time of less than 10 minutes, provided that the dosage form does not contain a calcium salt of ibuprofen, in the manufacture of a medicament to treat pain and/or fever. An onset-hastened analgesic and/or anti-pyretic response may be obtained.

The preparation of compressed tablets from formulations of the present invention is illustrated by the following Examples. In the Examples the racemic ibuprofen and racemic ibuprofen sodium salt is available from Knoll Pharma, Nottingham, GB; the grades of microcrystalline cellulose are available from the FMC Corporation, Brussels, BE under the tradenames Avicel PH101 and PH102; Croscarmellose sodium is available from the FMC Corporation, Brussels, BE under the tradename Ac-Di-Sol; colloidal silicon dioxide is available from Degussa, Frankfurt, DE under the tradename Aerosil 200; hydrogenated vegetable oil is available from Karlshamn, SE under the tradename Sterotex; hydroxypropylmethyl cellulose 2910 (50CPs) is available from Colorcon, Kent, GB; hydroxypropylmethyl cellulose 2910 (6CPs) is available from Shin-etsu, Japan and the Opaspray is available from Colorcon, Kent, GB; sodium starch glycollate is available from Edward Mendell, Reigate, GB, under the tradename Explotab; sodium stearyl fumarate is available from Forum Chemicals, Surrey, GB, under the tradename Pruv; mannitol is available from Roquette Freres, Lestrem, France, under the tradename Pearlitol, cross-linked polyvinylpyrrolidone is available from BASF, Ludwigshaven, Germany under the tradename Kollidon CL.

### A. Method of Preparation of Tablets in the Examples

The tablets were prepared by screening all the ingredients and blending until an homogenous mixture was obtained using a conventional blending machine. The formulation was then fed into and compressed on a single punch tabletting machine (Manesty F) using a compression force in the range 100 to 140 MPa. In some Examples, (Examples 1-9, 22) the compositions were compressed at particular compression forces, eg 100, 120, 140 MPa. In other Examples (Examples 10-21, 23-26) the compositions were compressed at an appropriate compression force within the range 100-140 MPa having regard to the ingredients used and the crushing strength and dissolution time required of the finished tablet.

### B. Measurement of the Properties of the Tablets Prepared in the Examples

### 1. Crushing Strength (Kp or N)

The crushing strength is a measure of the hardness of a tablet. It was measured by recording the diametrical crushing strength when the tablet was broken between the motorised jaws of a Schleuniger crushing strength tester. The range of crushing strengths of five tablets prepared with each Example formulation is given and the mean crushing strengths for Examples 10-27 are also given. The crushing strength is also given in Newtons (N).

### 2. Disintegration Time (Minutes)

The disintegration time was measured using the disintegration method described in the European Pharmacopoeia 1986, Ref V.5.1.1 (updated 1995) using tap water (pH approximately 7) as the liquid. The method provides the time by which six tablets prepared with each Example formulation had all disintegrated.

### C. Example Tablets and Properties Thereof

% are given in weight
Ibuprofen is racemic ibuprofen

### Examples 1-3

| Ingredients | **Example 1** | **Example 2** | **Example 3** |
|---|---|---|---|
| Content of drug per tablet (mg) | 256mg | 256mg | 256mg |
| Ibuprofen sodium salt dihydrate | 51.2% | 53.1 % | 51.2% |
| Microcrystalline cellulose (PH 101) | - | 13.3% | 12.8% |
| Microcrystalline cellulose (PH 102) | 35.4% | - | - |
| Lactose NF (Spray Dried) | - | 14.9% | 8.0% |
| Anhydrous sodium carbonate | 5.0% | 10.4% | 20.0% |
| Croscarmellose sodium | 7.2% | 7.5% | 7.2% |
| Colloidal silicon dioxide | 0.2% | - | - |
| Stearic acid | 0.5% | 0.8% | 0.8% |
| Magnesium stearate | 0.5% | - | - |

| Properties of Tablet | **Example 1** | | |
|---|---|---|---|
| Compression force (MPa) | 100 | 120 | 140 |
| Crushing Strength Range (Kp) | 10.4-10.7 (101.9-104.9 N) | 10.7-11.5 (104.9-112.7 N) | 10.3-11.2 (100.9-109.8 N) |
| Disintegration Time (min) | 5.8 | 5.4 | 5.0 |

| Properties of Tablet | **Example 2** | | |
|---|---|---|---|
| Compression force (MPa) | 100 | 120 | 140 |
| Crushing Strength Range (Kp) | 8.8-9.2 (86.2-90.2 N) | 7.2-10.8 (70.6-105.8 N) | 9.3-11.0 (91.1-107.8 N) |
| Disintegration Time (min) | 3.5 | 3.5 | 4.5 |

| Properties of Tablet | **Example 3** | | |
|---|---|---|---|
| Compression force (MPa) | 100 | 120 | 140 |
| Crushing Strength Range (Kp) | 8.5-9.5 (83.3-93.1 N) | 9.3-10.4 (91.1-101.9 N) | 11.1-11.7 (108.8-114.7 N) |
| Disintegration Time (min) | 4.3 | 4.7 | 4.9 |

### Examples 4-6

| **Ingredients** | **Example 4** | **Example 5** | **Example 6** |
|---|---|---|---|
| Content of drug per tablet (mg) | 256mg | 256mg | 256mg |
| Ibuprofen sodium salt dihydrate | 53.1% | 53.1% | 51.2% |
| Microcrystalline cellulose (PH 101) | 13.3% | 13.3% | 12.8% |
| Lactose NF (Spray Dried) | 14.9% | 14.9% | 14.4% |
| Anhydrous sodium carbonate | 10.4% | 10.4% | 10.0% |
| Croscarmellose sodium | 7.5% | 7.5% | 7.2% |
| Stearic acid | - | - | 0.8% |
| Magnesium stearate | 0.8% | - | - |
| Hydrogenated Vegetable Oil | - | 0.8% | - |
| Talc | - | - | 3.6% |

| Properties of Tablet | **Example 4** | | |
|---|---|---|---|
| Compression force (MPa) | 100 | 120 | 140 |
| Crushing Strength Range (Kp) | 6.6-7.2 (64.7-70.6 N) | 8.3-10.2 (81.3-100.0 N) | 8.8-10.1 (86.2-99.0 N) |
| Disintegration Time (min) | 4.7 | 5.4 | 5.3 |

| Properties of Tablet | **Example 5** | | |
|---|---|---|---|
| Compression force (MPa) | 100 | 120 | 140 |
| Crushing Strength Range (Kp) | 6.6-6.9 (64.7-67.6 N) | 8.5-9.1 (83.3-89.2 N) | 9.0-10.7 (88.2-104.9 N) |
| Disintegration Time (min) | 2.9 | 3.2 | 3.7 |

| Properties of Tablet | **Example 6** | | |
|---|---|---|---|
| Compression force (MPa) | 100 | 120 | 140 |
| Crushing Strength Range (Kp) | 8.1-8.6 (79.4-84.3 N) | 9.7-10.5 (95.1-102.9 N) | 10.7-11.6 (104.9 -113.7 N) |
| Disintegration Time (min) | 3.5 | 3.9 | 4.5 |

### Examples 7-9

| **Ingredients** | **Example 7** | **Example 8** | **Example 9** |
|---|---|---|---|
| **Content of drug per tablet (mg)** | **256mg** | **256mg** | **256mg** |
| Ibuprofen sodium salt dihydrate | 51.2% | 51.2% | 51.2% |
| Microcrystalline cellulose (PH 101) | 27.2% | - | - |
| Microcrystalline cellulose (PH 102) | - | 35.4% | 29.6% |
| Anhydrous sodium carbonate | 10.0% | 5.0% | 10.0% |
| Croscarmellose sodium | 7.2% | 7.2% | 7.2% |
| Colloidal silicon dioxide | - | 0.2% | 1.0% |
| Stearic acid | 1.0% | 1.0% | 0.5% |
| Magnesium stearate | - | - | 0.5% |
| Talc | 3.4% | - | - |

| Properties of Tablet | **Example 7** | | |
|---|---|---|---|
| Compression force (MPa) | 100 | 120 | 140 |
| Crushing Strength Range (Kp) | 7.0-7.4 (68.6-72.5 N) | 8.1-9.1 (79.4-89.2 N) | 7.9-10.4 (77.4-101.9 N) |
| Disintegration Time (min) | 3 | 3.8 | 4.5 |

| Properties of Tablet | **Example 8** | | |
|---|---|---|---|
| Compression force (MPa) | 100 | 120 | 140 |
| Crushing Strength Range (Kp) | 8.4-9.1 (82.3-89.2 N) | 10.1-10.6 (99.0-103.9 N) | 12.2-12.7 (119.6-124.5 N) |
| Disintegration Time (min) | 3.1 | 4.1 | 4.8 |

| Properties of Tablet | **Example 9** | | |
|---|---|---|---|
| Compression force (MPa) | 100 | 120 | 140 |
| Crushing Strength Range (Kp) | 5.8-6.2 (56.8-60.8 N) | 7.3-7.9 (71.5-77.4 N) | 9.2-9.8 (90.2-96.0 N) |
| Disintegration Time (min) | 2.2 | 3.3 | 4.7 |

### Examples 10 and 11

| **Ingredients** | **Example 10** | **Example 11** |
|---|---|---|
| **Content of drug per tablet (mg)** | **256mg** | **256mg** |
| Ibuprofen sodium salt dihydrate | 49.7% | 51.2% |
| Microcrystalline cellulose (PH 101) | - | 12.8% |
| Microcrystalline cellulose (PH 102) | 34.3% | - |
| Lactose | - | 8.0% |
| Anhydrous sodium carbonate | 7.8% | - |
| Sodium bicarbonate BP | - | 20.0% |
| Croscarmellose sodium | 7.0% | 7.2% |
| Colloidal silicon dioxide | 0.2% | - |
| Stearic acid | 1.0% | 0.8% |

| Properties of Tablet | **Example 10** | **Example 11** |
|---|---|---|
| Compression force (MPa) | 100-140 | 100-140 |
| Crushing Strength Range (Kp) | 7.1-8.0 (69.6-78.4 N) | 8.2-9.2 (80.4-90.2 N) |
| Mean Crushing Strength (Kp) | 7.5 (73.5 N) | 8.8 (86.2 N) |
| Disintegration Time (min) | 4.3 | 6.0 |

The tablet core of Example 10 was coated with the following coatings (% are given of core weight):-
First coat: hydroxypropylmethyl cellulose 2910 (6Cps) (1.016%), talc (0.204%), Opaspray (RTM) White M-I-7111B (0.336%).
Outer coat: hydroxypropylmethylcellulose 2910 (5-0Cps) (0.437%), Polyethylene Glycol 6000 (0.049%), calcium stearate (0.002%).

The disintegration time of the coated tablet of Example 10 was 5.5 minutes.

### Examples 12-14

| **Ingredients** | **Example 12** | **Example 13** | **Example 14** |
|---|---|---|---|
| **Content of drug per tablet (mg)** | **256mg** | **256mg** | **256mg** |
| Ibuprofen sodium salt dihydrate | 51.7% | 49.7% | 49.7% |
| Microcrystalline cellulose (PH 102) | 35.7% | 34.3% | 34.3% |
| Anhydrous sodium carbonate | 4.0% | - | 7.8% |
| Sodium bicarbonate - BP | - | 7.8% | - |
| Croscarmellose sodium | 7.3% | 7.0% | - |
| Sodium starch glycollate | - | - | 7.0% |
| Colloidal silicon dioxide | 0.3% | 0.2% | 0.2% |
| Stearic acid | 1.0% | 1.0% | 1.0% |

| Properties of Tablet | **Example 12** | **Example 13** | **Example 14** |
|---|---|---|---|
| Compression force (MPa) | 100-140 | 100-140 | 100-140 |
| Crushing Strength range (Kp) | 7.7-9.1 (75.5-89.2 N) | 8.7-9.6 (85.3-94.1 N) | 5.7-7.1 (55.9-69.6 N) |
| Mean Crushing Strength (Kp) | 8.7 (85.3 N) | 9.1 (89.2 N) | 6.0 (58.8 N) |
| Disintegration Time (min) | 3.5 | 4.5 | 5.8 |

The tablet cores of Examples 12-14 had the same coatings applied as described in Example 10. The disintegration times were 5.1 min, 5.5 min and 7.5 mins respectively for Examples 12, 13 and 14.

### Examples 15-17

| **Ingredients** | **Example 15** | **Example 16** | **Example 17** |
|---|---|---|---|
| **Content of drug per tablet (mg)** | **256mg** | **256mg** | **256mg** |
| Ibuprofen sodium salt dihydrate | 51.7% | 49.7% | 51.2% |
| Microcrystalline cellulose (PH 102) | 35.7% | 34.3% | 35.4% |
| Anhydrous sodium bicarbonate | 4.0% | - | 5.0% |
| Sodium bicarbonate | - | 7.8% | - |
| Croscarmellose sodium | - | - | 7.2% |
| Sodium starch glycollate | 7.3% | 7.0% | - |
| Colloidal silicon dioxide | 0.3% | 0.2% | 0.2% |
| Stearic acid | 1.0% | 1.0% | - |
| Sodium stearyl fumarate | - | - | 1.0% |

| Properties of Tablet | **Example 15** | **Example 16** | **Example 17** |
|---|---|---|---|
| Compression force (MPa) | 100-140 | 100-140 | 100-140 |
| Crushing Strength Range (Kp) | 6.2-8.1 (60.8-79.4 N) | 6.4-7.2 (62.7-70.6 N) | 10.0-11.6 (98.0-113.7 N) |
| Mean Crushing Strength (Kp) | 6.9 (67.6 N) | 6.7 (65.7 N) | 10.7 (104.9 N) |
| Disintegration Time (min) | 5.5 | 4.9 | 4.8 |

### Examples 18-20

| **Ingredients** | **Example 18** | **Example 19** | **Example 20** |
|---|---|---|---|
| **Content of drug per tablet (mg)** | **256mg** | **256mg** | **256mg** |
| Ibuprofen sodium salt dihydrate | 50.7% | 51.2% | 51.2% |
| Microcrystalline cellulose (PH 101) | - | 12.8% | 12.8% |
| Microcrystalline cellulose (PH 102) | 35.0% | - | - |
| Lactose NF (Spray Dried) | - | 14.4% | 14.4% |
| Anhydrous sodium carbonate | 5.9% | 10.0% | 10.0% |
| Croscarmellose sodium | 7.1% | 7.2% | 7.2% |
| Colloidal silicon dioxide | 0.3% | - | - |
| Stearic acid | 1.0% | - | - |
| Hydrogenated Vegetable Oil | - | 1.6% | 1.0% |
| Talc | - | 2.8% | 3.4% |

| Properties of Tablet | **Example 18** | **Example 19** | **Example 20** |
|---|---|---|---|
| Compression force (MPa) | 100-140 | 100-140 | 100-140 |
| Crushing Strength Range (Kp) | 8.5-9.4 (83.3-92.1 N) | 10.0-10.8 (98.0-105.8 N) | 9.1-10.3 (89.2-100.9 N) |
| Mean Crushing Strength (Kp) | 8.9 (87.2 N) | 10.4 (101.9 N) | 9.7 (95.1 N) |
| Disintegration Time (min) | 4.8 | 3.9 | 5.7 |

### Examples 21-23

| **Ingredients** | **Example 21** | **Example 22** | **Example 23** |
|---|---|---|---|
| **Content of drug per tablet (mg)** | **256mg** | **256mg** | **200mg** |
| Ibuprofen sodium salt dihydrate | 51.2% | 49.7% | - |
| *Ibuprofen | - | - | 49.7% |
| Microcrystalline cellulose (PH 101) | 12.8% | - | - |
| Microcrystalline cellulose (PH 102) | - | 34.3% | 34.3% |
| Mannitol 300 | 14.4% | - | - |
| Anhydrous sodium carbonate | 10.0% | 7.7% | 7.8% |
| Croscarmellose sodium | 7.2% | 7.0% | 7.0% |
| Colloidal silicon dioxide | - | 0.3% | 0.2% |
| Stearic acid | 1.0% | 0.5% | 1.0% |
| Magnesium stearate | - | 0.5% | - |
| Talc | 3.4% | - | - |

| | | | |
|---|---|---|---|
| * 50µm crystal size | | | |

| Properties of Tablet | **Example 21** |
|---|---|
| Compression force (MPa) | 100-140 |
| Crushing Strength Range (Kp) | 8.9-9.7 (87.2-95.1 N) |
| Mean Crushing Strength (Kp) | 9.4 (92.1 N) |
| Disintegration Time (min) | 4.0 |

| Properties of Tablet | **Example 22** | | |
|---|---|---|---|
| Compression force (MPa) | 100 | 120 | 140 |
| Mean Crushing Strength (Kp) | 10.2 (100.0 N) | 10.5 (102.9 N) | 10.5 (102.9 N) |
| Disintegration Time (min) | 4.8 | 5.5 | 6.0 |

| Properties of Tablet | **Example 23** |
|---|---|
| Compression force (MPa) | 100-140 |
| Crushing Strength Range (Kp) | 6.6-7.0 (64.7-68.6 N) |
| Mean Crushing Strength (Kp) | 6.8 (66.6 N) |
| Disintegration Time (min) | 0.6 |

Examples may also be prepared in a similar way to Examples 1-22 above containing the sodium salt of racemic ibuprofen in an amount of 64mg, 128mg, 192mg, 384mg, 512mg using the same proportions of ingredients as given in Examples 1-22.

### Examples 24-26

| **Ingredients** | **Example 24** | **Example 25** | **Example 26** |
|---|---|---|---|
| **Content of drug per tablet (mg)** | **342.0g** | **342.0g** | **342.0g** |
| Ibuprofen (dl lysine salt) | 68.4% | 49.7% | 49.7% |
| Microcrystalline cellulose (PH 102) | 20.35% | - | - |
| Hydroxypropylmethylcellulose | - | 34.3% | - |
| Tricalcium phosphate | - | - | 34.3% |
| Anhydrous sodium carbonate | 5.0% | 7.8% | 7.8% |
| Croscarmellose sodium | 5.0% | - | - |
| Cross-linked polyvinyl pyrrolidone | - | 7.0% | 7.0% |
| Colloidal silicon dioxide | 0.25% | 0.2% | 0.2% |
| Stearic acid | 1.0% | 1.0% | 1.0% |

| Properties of Tablet | **Example 24** | | |
|---|---|---|---|
| Compression force (MPa) | 100 | 120 | 140 |
| Crushing Strength (Kp) | 6.0 (58.8 N) | 7.0 (68.6 N) | 8.0 (78.4 N) |
| Disintegration Time (min) | 4.0 | 4.5 | 4.8 |

| Properties of Tablet | **Example 25** | **Example 26** |
|---|---|---|
| Compression force (MPa) | 100-140 | 100-140 |
| Crushing Strength Range (Kp) | 9.0-13.8 (88.2-135.2 N) | 10.5-10.8 (102.9-105.8 N) |
| Mean Crushing Strength (Kp) | 11.3 (110.7 N) | 10.6 (103.9 N) |
| Disintegration Time (min) | 8.0 | 7.5 |

Tablets may also be prepared in a similar manner to Examples 24-26 above containing the ibuprofen dl lysine salt in an amount of 171.0mg, 256.5mg and 513.0mg using the same proportions of ingredients as given in Examples 24-26.

### Comparative Examples

### A. Tablets containing 256mg racemic ibuprofen sodium salt

### (Ibuprofen equivalent 200mg)

| Ingredient | Comparative Formulation A (without (bi)carbonate component) |
|---|---|
| | % (wt) |
| Ibuprofen sodium salt dihydrate | 53.9% |
| Microcrystalline cellulose (PH102) | 37.2% |
| Croscarmellose sodium | 7.6% |
| Colloidal silicon dioxide | 0.3% |
| Stearic acid | 0.5% |
| Magnesium stearate | 0.5% |

### B. Tablets containing 342.0mg racemic ibuprofen lysine salt

### (Ibuprofen equivalent 200mg)

| Ingredient | Comparative Formulation B (without (bi)carbonate component) |
|---|---|
| | % (wt) |
| Ibuprofen (dl lysine salt) | 69.9 |
| Microcrystalline cellulose (PH102) | 23.4 |
| Croscarmellose sodium | 5.3 |
| Colloidal silicon dioxide | 0.4 |
| Stearic acid | 1.0 |

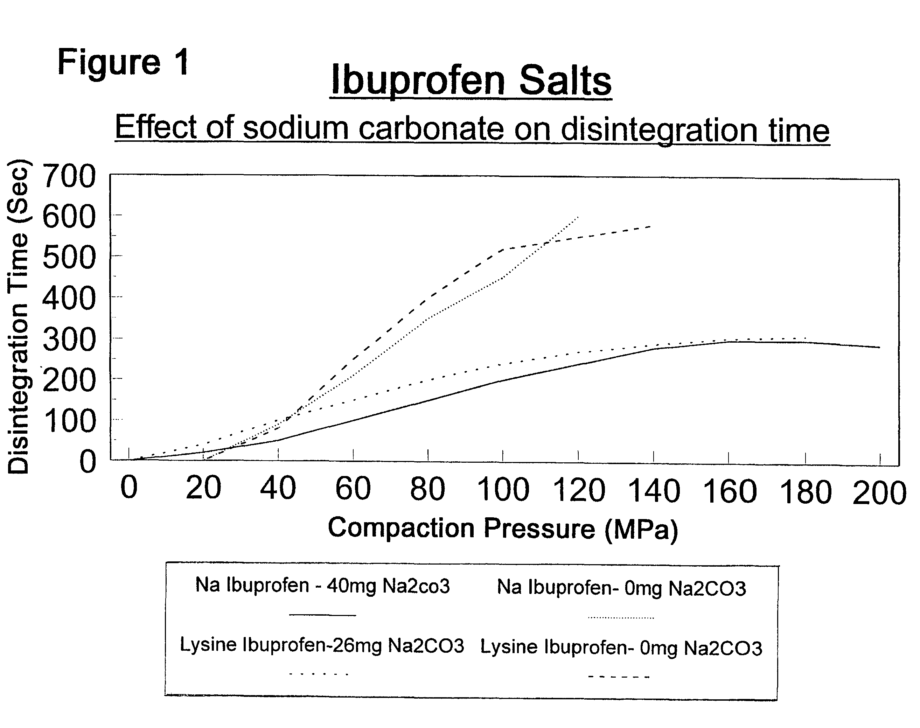

In the Figures, Figure 1 shows a comparison of the disintegration times of:-
(a) a compressed dosage form of the present invention containing the sodium salt of ibuprofen (Example 22) with comparative Example A (without a (bi)carbonate component); and
(b) a compressed dosage form of the present invention containing the lysine salt of ibuprofen (Example 24) with comparative Example B (without a (bi)carbonate component).

The disintegration times are shown as a function of compaction pressure.

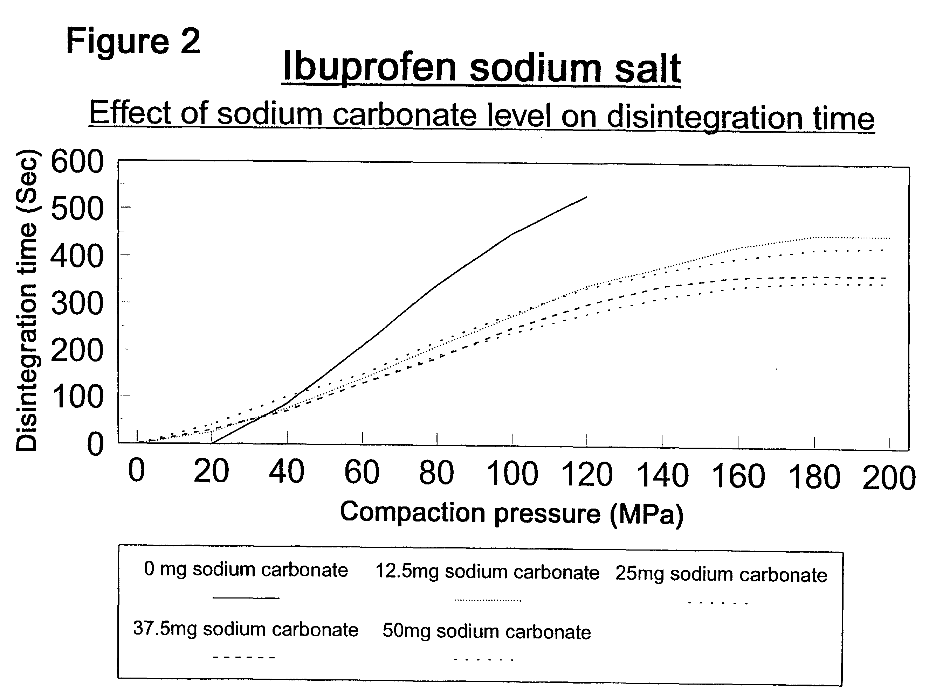

Figure 2 shows a comparison of the disintegration properties of the tablets having the following components with no sodium carbonate (Comparative Formulation A) and varying amounts of sodium carbonate additionally included in that Example (as shown below). The disintegration time is shown as a function of the compaction pressure.

| **Ingredient** | **Comparative Formulation A wt (mg)** | **Ex 27 wt (mg)** |
|---|---|---|
| Ibuprofen sodium salt dihydrate | 256.00 | 256.00 |
| Microcrystalline cellulose (PH 102) | 176.75 | 176.75 |
| Anhydrous sodium carbonate | - | 12.50 |
| Croscarmellose sodium | 36.00 | 36.00 |
| Colloidal silicon dioxide | 1.25 | 1.25 |
| Stearic acid | 2.50 | 2.50 |
| Magnesium stearate | 2.50 | 2.50 |

| **Ingredient** | **Ex 28 wt (mg)** | **Ex 29 wt (mg)** | **Ex 30 wt (mg)** |
|---|---|---|---|
| Ibuprofen sodium salt dihydrate | 256.00 | 256.00 | 256.00 |
| Microcrystalline cellulose (PH 102) | 176.75 | 176.75 | 176.75 |
| Anhydrous sodium carbonate | 25.00 | 37.50 | 50.00 |
| Croscarmellose sodium | 36.00 | 36.00 | 36.00 |
| Colloidal silicon dioxide | 1.25 | 1.25 | 1.25 |
| Stearic acid | 2.50 | 2.50 | 2.50 |
| Magnesium stearate | 2.50 | 2.50 | 2.50 |

It can be seen from Figures 1 and 2 that at standard operating compaction pressures in the range 100-140MPa, the disintegration time of the tablet without sodium carbonate steeply rises reflecting a sharp increase in disintegration time for only a small increase in compaction pressure. The disintegration time vs compaction force gradient for tablets containing sodium carbonate is unexpectedly much more shallow which leads to the processing advantages described herein. In Figure 2 it can be seen that the disintegration times at 100MPa for tablets containing sodium carbonate are less than 300 seconds, whereas omitting this component provides a disintegration time greater than 420 seconds.

## Claims

1. A solid non-effervescent compressed dosage form comprising a racemic ibuprofen medicament and a carrier material comprising a compressible filler component combined with a disintegrating component wherein the ibuprofen medicament is present in an amount of 35% or more by weight of the dosage form, **characterised in that** the carrier material includes a solid alkali metal carbonate or bicarbonate present in an amount of 3-20% by weight of the dosage form, said solid alkali metal carbonate or bicarbonate being in homogeneous admixture with said ibuprofen medicament and said compressible filler component combined with the disintegrating component, such that the dosage form has a crushing strength in the range 63,7-147 N (6,5-15 Kp) and a disintegration time of less than 10 minutes as measured by the method described in the European Pharmacopoeia 1986, Ref. V.5.1.1., provided that the ibuprofen medicament does not contain a calcium salt of ibuprofen in combination with an alkali metal salt of ibuprofen.

2. A dosage form according to claim 1 wherein the ibuprofen medicament is in the form of a salt of racemic ibuprofen.

3. A dosage form according to claim 2 wherein the ibuprofen medicament is the sodium salt of racemic ibuprofen.

4. A dosage form according to any one of claims 1 to 3 comprising 10-50% by weight of the dosage form of the compressible filler component, up to 15% by weight of the dosage form of a discrete disintegrant component selected from one or more of wheat starch, maize starch, potato starch, sodium starch glycollate, low-substituted hydroxypropylcellulose, alginic acid, cross-linked polyvinylpyrrolidone, magnesium aluminium silicate and croscarmellose sodium and 0-10% by weight of the dosage form of an inert diluent component.

5. A dosage form according to any one of claims 1 to 4 comprising 5-15% alkali metal carbonate or bicarbonate by weight of the dosage form.

6. A dosage form according to any one of claims 1 to 5 wherein the alkali metal carbonate or bicarbonate comprises sodium carbonate or sodium bicarbonate.

7. A dosage form according to claim 6 comprising sodium carbonate or bicarbonate in a weight ratio to the ibuprofen medicament of 1:2 to 1:10.

8. A dosage form according to any one of claims 1 to 7 wherein the compressible filler component comprises one or more of microcrystalline cellulose, lactose and mannitol.

9. A dosage form according to any one of claims 1 to 8 wherein the disintegrating component comprises one or more of croscarmellose sodium and sodium starch glycollate.

10. A dosage form according to any one of claims 1 to 9 in the form of a compressed tablet.

11. The use of a non-effervescent compressed solid dosage form comprising 35% or more by weight of a sodium salt of racemic ibuprofen together with a carrier material comprising a compressible filler component combined with a disintegrating component and an alkali metal carbonate or bicarbonate present in an amount of 3-20% by weight of the dosage form, the dosage form having a crushing strength in the range 63,7-147 N (6,5-15 Kp) and a disintegration time of less than 10 minutes as measured by the method described in the European Pharmacopoeia 1986, Ref. V.5.1.1., provided that the dosage form does not contain a calcium salt of ibuprofen, in the manufacture of a medicament to treat pain and/or fever.

12. The use according to claim 11 wherein the carrier material is adapted for direct compression with the ibuprofen medicament into a tablet.

13. The use according to claim 11 or 12 wherein the solid dosage form comprises the sodium salt of ibuprofen together with a carrier material comprising microcrystalline cellulose and sodium carbonate or bicarbonate.

14. The use according to claims 11 to 13 wherein carrier material comprises 45-60% by weight microcrystalline cellulose, 2-10% by weight croscarmellose sodium and 2-20% by weight sodium carbonate or bicarbonate.

15. A use according to claim 11 wherein the dosage form has a crushing strength in the range 78,4-117,6 N (8-12 Kp) at a compression force in the range 100-140MPa.

16. A use according to either one of claims 11 and 15 wherein the solid dosage form has a disintegration time in the range 1-5 minutes.

17. A use according to any one of claims 11, 15 and 16 wherein the dosage form is in the form of a directly compressed tablet comprising 40-85% by weight sodium salt of ibuprofen and 5-15% by weight sodium carbonate or bicarbonate.

18. A process to prepare a non-effervescent solid dosage form comprising a racemic ibuprofen medicament present in an amount of 35% or more by weight of the dosage form **characterised by** combining a carrier material comprising a compressible filler component combined with a disintegrating component and 3-20% by weight of the dosage form of an alkali metal carbonate or bicarbonate with the ibuprofen medicament to form a homogeneous solid mixture under substantially dry conditions optionally with other tabletting excipients and compressing the mixture into one or more solid dosage forms having a crushing strength in the range 63,7-147 N (6,5-15 Kp) and a disintegration time of less than 10 minutes as measured by the method described in the European Pharmacopoeia 1986 ,Ref. V.5.1.1..

19. A process according to claim 18 wherein the ibuprofen medicament is a salt of racemic ibuprofen.

20. A process according to either one of claims 18 and 19 wherein the carrier material comprises an inert diluent component.

21. A process according to any one of claims 18 to 20 wherein the dosage form is prepared by direct compression of a powder mixture of the ingredients and does not include any pre-granulation stage.

22. A process according to any one of claims 19 to 21 wherein the ratio of the alkali metal carbonate or bicarbonate to compressible filler component is in the range 2:1 to 1:10 parts by weight.

23. A process according to any one of claims 19 to 22 wherein the ratio of ibuprofen medicament to the carrier material is in the range 2:1 to 1:2 parts by weight and the carrier material comprises 5-20% w/w sodium carbonate or bicarbonate.

24. A solid formulation having a layer comprising a composition comprising a racemic ibuprofen medicament together with a carrier material, the racemic ibuprofen medicament being present in an amount of 35% or more by weight of the composition and the carrier material comprising a compressible filler component combined with a disintegrating component **characterised in that** the carrier material comprises a solid alkali metal carbonate or bicarbonate present in an amount of 3-20% by weight of the composition, said solid alkali metal carbonate or bicarbonate being in homogeneous admixture with said ibuprofen medicament and said compressible filler component combined with disintegrating component such that the composition is capable of compression to provide a layer having a crushing strength in the range 63,7-147 N (6,5-15 Kp) and a disintegration time of less than 10 minutes as measured by the method described in the European Pharmacopoeia 1986, Ref V.5.1.1..

## Patentansprüche

1. Feste, nicht aufbrausende, komprimierte Dosierungsform enthaltend ein racemisches Ibuprofen Medikament und ein Trägermaterial, das eine komprimierbare Füllkomponente in Kombination mit einer Sprengkomponente umfaßt, wobei das Ibuprofen Medikament in einer Menge von 35 Gew.-% oder mehr der Dosierungsform vorhanden ist, **dadurch gekennzeichnet, daß** das Trägermaterial ein festes Alkalicarbonat oder -hydrogencarbonat einschließt, das in einer Menge von 3-20 Gew.-% der Dosierungsform vorliegt, wobei sich das feste Alkalicarbonat bzw. -hydrogencarbonat in einer homogenen Mischung mit dem Ibuprofen Medikament befindet und die komprimierbare Füllkomponente mit der Sprengkomponente kombiniert ist, so daß die Dosierungsform eine Druckfestigkeit im Bereich von 63,7-147 N (6,5-15 Kp) und eine Zerfallzeit von weniger als 10 Minuten (gemessen durch das in der europäischen Pharmakopöe 1986, Ref. V. 5.1.1. beschriebene Verfahren) hat, mit der Maßgabe, daß das Ibuprofen Medikament nicht ein Calciumsalz von Ibuprofen in Kombination mit einem Alkalimetallsalz von Ibuprofen enthält.

2. Dosierungsform nach Anspruch 1, wobei das Ibuprofen Medikament in Form eines Salzes von racemischem Ibuprofen vorliegt.

3. Dosierungsform nach Anspruch 2, wobei es sich bei dem Ibuprofen Medikament um das Natriumsalz von racemischem Ibuprofen handelt.

4. Dosierungsform nach einem der Ansprüche 1 bis 3, enthaltend 10-50 Gew.-% der Dosierungsform an der komprimierbaren Füllkomponente, bis zu 15 Gew.-% der Dosierungsform an einer davon verschiedenen Sprengkomponente, ausgewählt aus einer oder mehreren der folgenden Substanzen: Weizenstärke, Maisstärke, Kartoffelstärke, Natriumstärkeglycolat, Hydroxypropylcellulose mit niedrigem Substitutionsgrad, Algensäure, quervernetztem Polyvinylpyrrolidon, Magnesiumaluminiumsilikat und Croscarmellose-Natrium, und 0-10 Gew.-% der Dosierungsform an einer inerten Verdünnungskomponente.

5. Dosierungsform nach einem der Ansprüche 1 bis 4, enthaltend 5-15 Gew.-% der Dosierungsform an einem Alkalicarbonat oder -hydrogencarbonat.

6. Dosierungsform nach einem der Ansprüche 1 bis 5, wobei es sich bei dem Alkalicarbonat bzw. -hydrogencarbonat um Natriumcarbonat bzw. Natriumhydrogencarbonat handelt.

7. Dosierungsform nach Anspruch 6, enthaltend Natriumcarbonat bzw. -hydrogencarbonat in einem Gewichtsverhältnis zum Ibuprofen Medikament von 1:2 bis 1:10.

8. Dosierungsform nach einem der Ansprüche 1 bis 7, wobei die komprimierbare Füllkomponente eine oder mehrere der folgenden Substanzen enthält: mikrokristalline Cellulose, Lactose und Mannit.

9. Dosierungsform nach einem der Ansprüche 1 bis 8, wobei die Sprengkomponente eine oder mehrere der folgenden Substanzen enthält: Croscarmellose-Natrium und Natriumstärkeglycolat.

10. Dosierungsform nach einem der Ansprüche 1 bis 9 in Form einer komprimierten Tablette.

11. Verwendung einer nicht aufbrausenden, komprimierten, festen Dosierungsform, enthaltend 35 Gew.-% oder mehr eines Natriumsalzes von racemischem Ibuprofen zusammen mit einem Trägermaterial, umfassend eine komprimierbare Füllkomponente in Kombination mit einer Sprengkomponente und einem Alkalicarbonat oder -hydrogencarbonat in einer Menge von 3-20 Gew.-% der Dosierungsform, wobei die Dosierungsform eine Druckfestigkeit im Bereich von 63,7-147 N (6,5-15 Kp) und eine Zerfallszeit von weniger als 10 Minuten (gemessen durch das in der europäischen Pharmakopöe 1986, Ref. V.5.1.1. beschriebene Verfahren) hat, mit der Maßgabe, daß die Dosierungsform kein Calciumsalz von Ibuprofen enthält, zur Herstellung eines Medikaments zur Behandlung von Schmerzen und/oder Fieber.

12. Verwendung nach Anspruch 11, wobei das Trägermaterial für das direkte Verpressen mit dem Ibuprofen Medikament zu einer Tablette geeignet ist.

13. Verwendung nach Anspruch 11 oder 12, wobei die feste Dosierungsform das Natriumsalz von Ibuprofen zusammen mit einem Trägermaterial, umfassend mikrokristalline Cellulose und Natriumcarbonat oder -hydrogencarbonat, enthält.

14. Verwendung nach einem der Ansprüche 11 bis 13, wobei das Trägermaterial 45-60 Gew.-% mikrokristalline Cellulose, 2-10 Gew.-% Croscarmellose-Natrium und 2-20 Gew.-% Natriumcarbonat oder - hydrogencarbonat umfaßt.

15. Verwendung nach Anspruch 11, wobei die Dosierungsform eine Druckfestigkeit im Bereich von 78,4-117,6 N (8-12 Kp) bei einer Kompressionskraft im Bereich von 100-140 MPa aufweist.

16. Verwendung nach Anspruch 11 oder 15, wobei die feste Dosierungsform eine Zerfallszeit im Bereich von 1-5 Minuten aufweist.

17. Verwendung nach einem der Ansprüche 11, 15 und 16, wobei die Dosierungsform in Form einer direkt komprimierten Tablette vorliegt, die 40-85 Gew.-% des Natriumsalzes von Ibuprofen und 5-15 Gew.-% Natriumcarbonat oder -hydrogencarbonat enthält.

18. Verfahren zur Herstellung einer nicht aufbrausenden, festen Dosierungsform, enthaltend ein racemisches Ibuprofen Medikament in einer Menge von 35 Gew.-% oder mehr der Dosierungsform, **dadurch gekennzeichnet, daß** man ein Trägermaterial, das eine komprimierbare Füllkomponente in Kombination mit einer Sprengkomponente und 3-20 Gew.-% der Dosierungsform an einem Alkalicarbonat oder -hydrogencarbonat umfaßt, mit dem Ibuprofen Medikament unter Bildung einer homogenen festen Mischung unter wesentlichen trockenen Bedingungen, gegebenenfalls mit anderen Tablettierhilfsstoffen, kombiniert und die Mischung zu einer oder mehreren festen Dosierungsformen mit einer Druckfestigkeit im Bereich von 63,7-147 N (6,5-15 Kp) und einer Zerfallszeit von weniger als 10 Minuten (gemessen durch das in der europäischen Pharmakopöe 1986, Ref. V.5.1.1. beschriebene Verfahren) komprimiert.

19. Verfahren nach Anspruch 18, wobei es sich bei dem Ibuprofen Medikament um ein Salz von racemischem Ibuprofen handelt.

20. Verfahren nach Anspruch 18 oder 19, wobei das Trägermaterial eine inerte Verdünnungskomponente umfaßt.

21. Verfahren nach einem der Ansprüche 18 bis 20, wobei man die Dosierungsform herstellt, indem man eine Pulvermischung der Bestandteile direkt, ohne Vorgranulierungsstufe, komprimiert.

22. Verfahren nach einem der Ansprüche 19 bis 21, wobei das Verhältnis an Alkalicarbonat bzw. - hydrogencarbonat zu komprimierbarer Füllkomponente im Bereich von 2:1 bis 1:10 Gew.-Teilen liegt.

23. Verfahren nach einem der Ansprüche 19 bis 22, wobei das Verhältnis von Ibuprofen Medikament zum Trägermaterial im Bereich von 2:1 bis 1:2 Gew.-Teilen liegt und das Trägermaterial 5-20 Gew.-% Natriumcarbonat bzw. -hydrogencarbonat enthält.

24. Feste Formulierung, die eine Schicht aufweist, die eine Zusammensetzung enthält, die ein racemisches Ibuprofen Medikament zusammen mit einem Trägermaterial enthält, wobei das racemische Ibuprofen Medikament in einer Menge von 35 Gew.-% oder mehr der Zusammensetzung vorhanden ist und das Trägermaterial eine komprimierbare Füllkomponente in Kombination mit einer Sprengkomponente umfaßt, **dadurch gekennzeichnet, daß** das Trägermaterial ein festes Alkalicarbonat oder -hydrogencarbonat enthält, das in einer Menge von 3-20 Gew.-% der Zusammensetzung vorhanden ist, wobei das feste Alkalicarbonat bzw. - hydrogencarbonat sich in einer homogenen Mischung mit dem Ibuprofen Medikament befindet und die komprimierbare Füllkomponente mit der Sprengkomponente kombiniert ist, so daß die Zusammensetzung unter Bereitstellung einer Schicht mit einer Druckfestigkeit im Bereich von 63,7-147 N (6,5-15 Kp) und einer Zerfallszeit von weniger als 10 Minuten (gemessen nach dem in der europäischen Pharmakopöe 1986, Ref. V.5.1.1. beschriebene Verfahren) komprimiert werden kann.

## Revendications

1. Forme posologique comprimée non effervescente solide comprenant un médicament ibuprofène racémique et un véhicule comprenant un constituant de charge compressible associé à un constituant délitant, dans laquelle le médicament ibuprofène est présent à raison de 35% en poids ou plus de la forme posologique, **caractérisée en ce que** le véhicule contient un carbonate ou bicarbonate de métal alcalin solide présent à raison de 3-20% en poids de la forme posologique, ledit carbonate ou bicarbonate de métal alcalin solide étant en mélange homogène avec ledit médicament ibuprofène et ledit constituant de charge compressible associé au constituant délitant, de telle sorte que la forme posologique ait une résistance à l'écrasement dans la gamme de 63,7-147 N (6,5-15 Kp) et un temps de désagrégation inférieur à 10 minutes, mesuré par la méthode décrite dans la Pharmacopée Européenne 1986, réf. V.5.1.1., pourvu que le médicament ibuprofène ne contienne pas de sel de calcium d'ibuprofène associé à un sel de métal alcalin d'ibuprofène.

2. Forme posologique selon la revendication 1 dans laquelle le médicament ibuprofène est sous forme de sel d'ibuprofène racémique.

3. Forme posologique selon la revendication 2 dans laquelle le médicament ibuprofène est le sel de sodium d'ibuprofène racémique.

4. Forme posologique selon l'une quelconque des revendications 1 à 3 comprenant 10-50%, en poids de la forme posologique, du constituant de charge compressible, jusqu'à 15%, en poids de la forme posologique, d'un constituant délitant discret choisi parmi un ou plusieurs des produits amidon de blé, amidon de maïs, fécule de pomme de terre, glycolate sodique d'amidon, hydroxypropylcellulose à faible substitution, acide alginique, polyvinylpyrrolidone réticulée, aluminosilicate de magnésium et croscarmellose sodique, et 0-10%, en poids de la forme posologique, d'un constituant diluant inerte.

5. Forme posologique selon l'une quelconque des revendications 1 à 4 comprenant 5-15% de carbonate ou bicarbonate de métal alcalin, en poids de la forme posologique.

6. Forme posologique selon l'une quelconque des revendications 1 à 5 dans laquelle le carbonate ou bicarbonate de métal alcalin comprend le carbonate de sodium ou le bicarbonate de sodium.

7. Forme posologique selon la revendication 6 comprenant du carbonate ou bicarbonate de sodium dans un rapport pondéral par rapport au médicament ibuprofène de 1:2 à 1:10.

8. Forme posologique selon l'une quelconque des revendications 1 à 7 dans laquelle le constituant de charge compressible comprend un ou plusieurs des produits cellulose microcristalline, lactose et mannitol.

9. Forme posologique selon l'une quelconque des revendications 1 à 8 dans laquelle le constituant délitant comprend un ou plusieurs des produits croscarmellose sodique et glycolate sodique d'amidon.

10. Forme posologique selon l'une quelconque des revendications 1 à 9 sous forme de comprimé préparé par compression.

11. Utilisation d'une forme posologique solide comprimée non effervescente comprenant 35% en poids ou plus d'un sel de sodium d'ibuprofène racémique en même temps qu'un véhicule comprenant un constituant de charge compressible associé à un constituant délitant, et un carbonate ou bicarbonate de métal alcalin à raison de 3-20% en poids de la forme posologique, la forme posologique ayant une résistance à l'écrasement dans la gamme de 63,7-147 N (6,5-15 Kp) et un temps de désagrégation inférieur à 10 minutes, mesuré par la méthode décrite dans la Pharmacopée européenne 1986, réf. V.5.1.1., pourvu que la forme posologique ne contienne pas de sel de calcium d'ibuprofène, dans la fabrication d'un médicament destiné à traiter la douleur et/ou la fièvre.

12. Utilisation selon la revendication 11 dans laquelle le véhicule est conçu pour la compression directe en comprimé avec le médicament ibuprofène.

13. Utilisation selon la revendication 11 ou 12 dans laquelle la forme posologique solide comprend le sel de sodium d'ibuprofène en même temps qu'un véhicule comprenant de la cellulose microcristalline et du carbonate ou bicarbonate de sodium.

14. Utilisation selon les revendications 11 à 13 dans laquelle le véhicule comprend 45-60% en poids de cellulose microcristalline, 2-10% en poids de croscarmellose sodique et 2-20% en poids de carbonate ou bicarbonate de sodium.

15. Utilisation selon la revendication 11 dans laquelle la forme posologique a une résistance à l'écrasement dans la gamme de 78,4-117,6 N (8-12 Kp) sous une force de compression dans la gamme de 100-140 MPa.

16. Utilisation selon l'une quelconque des revendications 11 et 15 dans laquelle la forme posologique solide a un temps de désagrégation dans la gamme de 1-5 minutes.

17. Utilisation selon l'une quelconque des revendications 11, 15 et 16 dans laquelle la forme posologique est sous forme de comprimé préparé par compression directe comprenant 40-85% en poids de sel de sodium d'ibuprofène et 5-15% en poids de carbonate ou bicarbonate de sodium.

18. Procédé de préparation d'une forme posologique solide non effervescente comprenant un médicament ibuprofène racémique présent à raison de 35% en poids ou plus de la forme posologique, **caractérisé en ce que** l'on associe un véhicule comprenant un constituant de charge compressible à un constituant délitant et 3-20%, en poids de la forme posologique, d'un carbonate ou bicarbonate de métal alcalin avec le médicament ibuprofène pour former un mélange solide homogène dans des conditions essentiellement sèches, éventuellement avec d'autres excipients de fabrication de comprimés et **en ce que** l'on compresse le mélange en une ou plusieurs formes posologiques solides ayant une résistance à l'écrasement dans la gamme de 63,7-147 N (6,5-15 Kp) et un temps de désagrégation inférieur à 10 minutes, mesuré par la méthode décrite dans la Pharmacopée Européenne 1986, réf. V.5.1.1.

19. Procédé selon la revendication 18 dans lequel le médicament ibuprofène est un sel d'ibuprofène racémique.

20. Procédé selon l'une quelconque des revendications 18 et 19 dans lequel le véhicule comprend un constituant diluant inerte.

21. Procédé selon l'une quelconque des revendications 18 à 20 dans lequel la forme posologique est préparée par compression directe d'un mélange en poudre des ingrédients et ne comprend aucune étape de pré-granulation.

22. Procédé selon l'une quelconque des revendications 19 à 21 dans lequel le rapport du carbonate ou bicarbonate de métal alcalin au constituant de charge compressible est dans la gamme de 2:1 à 1:10 parties en poids.

23. Procédé selon l'une quelconque des revendications 19 à 22 dans lequel le rapport du médicament ibuprofène au véhicule est dans la gamme de 2:1 à 1:2 parties en poids et le véhicule comprend 5-20% en poids/poids de carbonate ou de bicarbonate de sodium.

24. Formulation solide comportant une couche comprenant une composition comprenant un médicament ibuprofène racémique en même temps qu'un véhicule, le médicament ibuprofène racémique étant présent à raison de 35% en poids ou plus de la composition et le véhicule comprenant un constituant de charge compressible associé à un constituant délitant, **caractérisée en ce que** le véhicule comprend un carbonate ou bicarbonate de métal alcalin solide présent à raison de 3-20% en poids de la composition, ledit carbonate ou bicarbonate de métal alcalin solide étant en mélange homogène avec ledit médicament ibuprofène et ledit constituant de charge compressible associé au constituant délitant, de telle sorte que la composition puisse être compressée pour donner une couche ayant une résistance à l'écrasement dans la gamme de 63,7-147 N (6,5-15 Kp) et un temps de désagrégation inférieur à 10 minutes, mesuré par la méthode décrite dans la Pharmacopée Européenne 1986, réf. V.5.1.1.
